# EUROPEAN PATENT APPLICATION

(11) **EP 1 034 809 A1**
(43) Date of publication of application: **13.09.2000**
(21) Application number: 99810168.7
(22) Date of filing: 26.02.1999
(51) Int. Cl.: A61M 5/30

(54) **Administering device for medication**

(71) Applicant: F. HOFFMANN-LA ROCHE AG, 4070 Basel (CH)
(72) Inventor: List, Martin, 4051 Basel (CH); Erni, Werner, 4106 Therwil (CH)
(74) Representative: AMMANN PATENTANWAELTE AG BERN

(57) **Abstract**

A method and a needleless injection system for administering a medication In order to simplify as far as possible the handling necessary for loading the injector with medication and thereby eliminate risks caused by a complex handling the method and system according to the invention provide loading of medication into a needleless injector device (11) by means of a syringe (13) prefilled with a medication (14), this loading step preferably concluding connecting the syringe (13) to the injector device (11) by means of a suitable coupling member or adapter (16) and operating the syringe (13) for transferring medication (14) prefilled therein into the injector device (11). After this transfer the syringe (13) and/or the coupling member or adapter (16) are detached from the injector device (11), which is then ready to be operated for ejecting the loaded dosage of said medication (14) from the injector device and thereby pass the selected dosage through the skin cf an injection receiver.

## Description

The invention concerns a method for parenterally administering a medication.

The invention also concerns a needleless injection system for administering a medication.

The International Patent Application published under No. WO 89/08469 describes a method and a system of the above mentioned kind. This known system comprises a needleless injector device, a vial coupler device and a transporter/loader device. The method of use of this known system comprises the following steps:
In a first step, one end of the vial coupler device has to be connected to a vial containing the medication to be administered, and this includes piercing a piercable closure of the vial with a cannula forming part of the vial coupler device.
In a second step, the opposite end of the vial coupler device has to be connected to the transporter/loader device in order to establish a fluidic connection between the interior of the vial and the interior of the transporter/loader device.
In a third step, the transporter/loader device has to be operated for transferring a certain amount of medication from the vial into the transporter/loader device, said amount passing through the vial coupler device.
In a fourth step the transporter/loader device has to be disconnected from the vial coupler and connected to the needleless injector device for transferring a certain amount of medication from the transporter/loader device to the needleless injector device.
In a fifth step the transporter/loader device has to be disconnected from the needleless injector device. Only after completion of this step is the needleless injector device ready for the intended use, that is, for administering the medication.

From the foregoing description of the method of use of the device described in the International Patent Application WO 89/08469, it can appreciated that these known method and system have the serious disadvantage that their use requires a complicated handling comprising several steps, and that corresponding risks result therefrom, in particular the risk of microbial contamination during the handling. Also misdosing (inaccurate amount of medication) is likely to occur.

The aim of the invention is therefore to provide a method and system for administering a medication and a needleless injection system which eliminate the above mentioned disadvantages, that is, to provide a method and system which make possible to load a needleless injection system with a minimum of handling, and thereby preventing the above mentioned risks.

According to a first aspect of the invention this aim is attained with a method according to claim 1.

According to a second aspect of the invention this aim is attained with a needleless injection system according to claim 3.

A preferred embodiments of the method according to the invention is defined by claim 2. Preferred embodiments of a needleless injection system according to the invention is defined by claim 4.

The main advantage of the method and the injection system according to the invention is that they make possible a completely needleless loading of the needleless injector device with medication prefilled in the syringe in a most simple way which requires a minimum of handling and therefore prevents risks resulting from complicate handling necessary with prior art devices and methods.

Preferred embodiments of the invention is described hereinafter with reference to the accompanying drawings wherein:
Fig. 1 schematically shows components of a preferred embodiment of a needleless injection system according to the invention,
Fig. 2 shows separately the end part of the injector device 11 in Fig. 1 and a coupling member 16, which is suitable for connecting that end part to the syringe 13 in Fig. 1,
Fig. 3 shows a schematic representation of the connection of injector device 11 and the syringe 13 by means of the coupling member 16, before the syringe is operated for transferring the medication prefilled therein into the injector device 11,
Fig. 4 shows a schematic representation of the connection of injector device 11 and the syringe 13 by means of the coupling member 16, after the syringe has been operated and the medication prefilled therein has been thereby transferred into the injector device 11,
Fig. 5 shows the syringe 13 and the coupling member 16 connected thereto after they have been detached from the injector device 11 after the transfer of the medication from the syringe into the injector device 11,
Fig. 6 shows the injector device 11 ready for use after it has been loaded with the medication and has been detached from the coupling member 16 and the syringe 13 connected thereto.

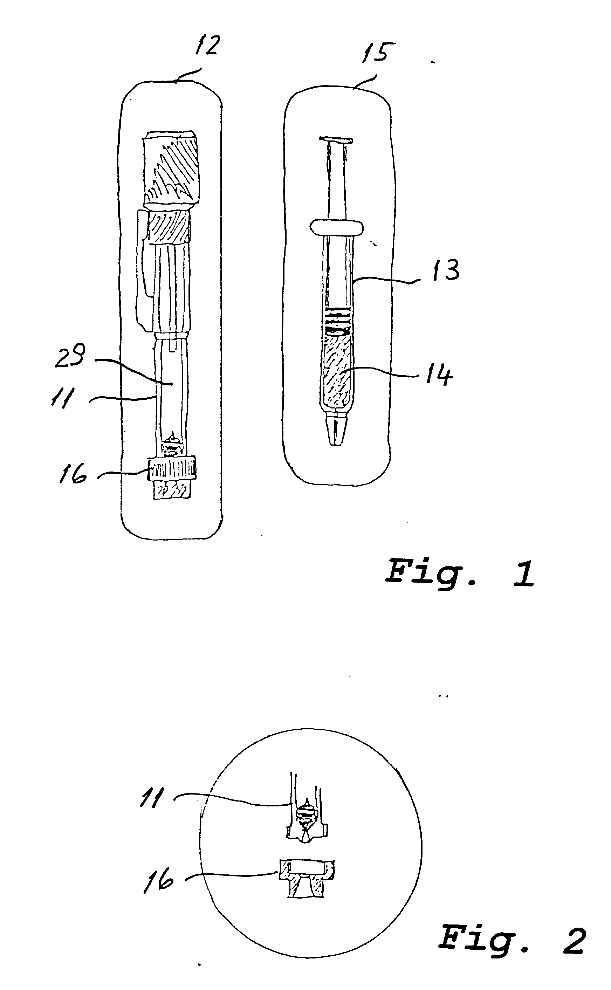

As schematically represented in Fig. 1 a needleless injection system according to the invention comprises the following components:
- a needleless injector device 11 that is dimensioned and arranged as an integral unit to be grasped in the hand of a user, the device 11 having a barrel portion, a gas storage portion, and an intermediate portion extending between the barrel portion and the gas storage portion; and
- a syringe 13 prefilled with a medication 14, said syringe being suitable for loading the injector device 11 with said medication 14 prefilled in the syringe.

In a preferred embodiment an injection system according to the invention further comprises a coupling member or adapter 16 for connecting the prefilled syringe 13 to the needleless injector device 11 and thereby establishing a fluidic communication between the interior of the syringe 13 and the interior of the injector device 11 for the purpose of transferring a quantity of medication from the prefilled syringe 13 into the needleless injector device 11.

As can be appreciated, in particular from Fig. 2, the coupling member or adapter 16 has a shape, which on one side matches the shape of an end part of the injector device 11 and on the opposite side matches the shape of the luer tip of the syringe 13, so that this tip can simply be plugged in that side of the coupling member or adapter 16. The side of the coupling member or adapter 16 which matches the shape of the injector device 11 is preferably adapted to be connected to that end part of the injector device 11 by a screw connection. As can be further appreciated, in particular from Fig. 2, the coupling member or adapter 16 has an inner shape which makes it possible to establish a fluidic communication between the interior of the injector device 11 and the interior of the syringe 13 when they are connected with each other by means of the coupling member or adapter 16.

The components of an injection system according to the invention are preferably conserved in closed, suitable envelopes 12 and 15 respectively, which are only opened just before use of the components for their intended purpose.

The needleless injector device 11, which is schematically represented in the drawings of this patent application is for example of the type described in the above mentioned International Patent Application WO 89/08469. The pertinent detailed description of this device in that patent application is incorporated herein by reference. The barrel portion of the injector device 11 has a chamber 29 for receiving medication to be injected by means of the injector device 11.

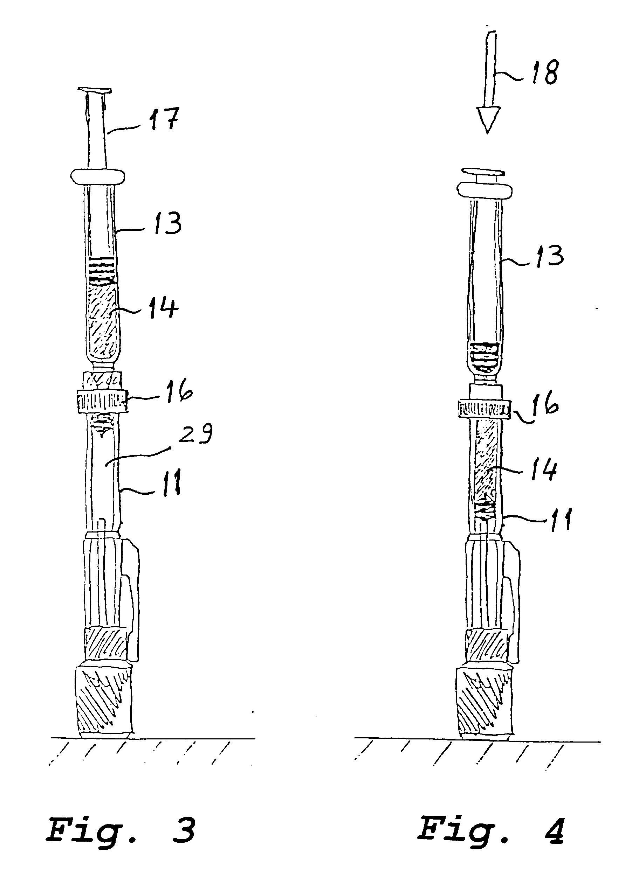

A preferred embodiment of a method according to the invention for administering a medication is described hereinafter in particular with reference to Figures 3 to 6.

To start with the user will of course first of all open the envelopes 12 and 15 and take therefrom the components of the injection system shown by Fig. 1. Then the user will screw the coupling member or adapter 16 on one end of the injector device 11 and he will plug the luer tip of the syringe 13 prefilled with a medication 14 into the coupling member or adapter 16. The resulting assembly of these components is shown by Fig. 3. It should be noted that this assembly establishes a fluidic communication between the interior of the syringe 13 and the interior of the injector device 11.

The next step is the transfer of the medication 14 prefilled in the syringe 13 into the chamber 29 of the barrel portion of the injector device 11 through an aperture of that barrel portion. For this purpose the user presses the plunger 17 of the syringe 13 in the sense indicated by arrow 18 in Fig. 4 and thereby forces the medication to flow out of the syringe 13, through the coupling member or adapter 16 and into the barrel of the injector device 11. At the end of this step the barrel of the injector device 11 is thus loaded with medication 14.

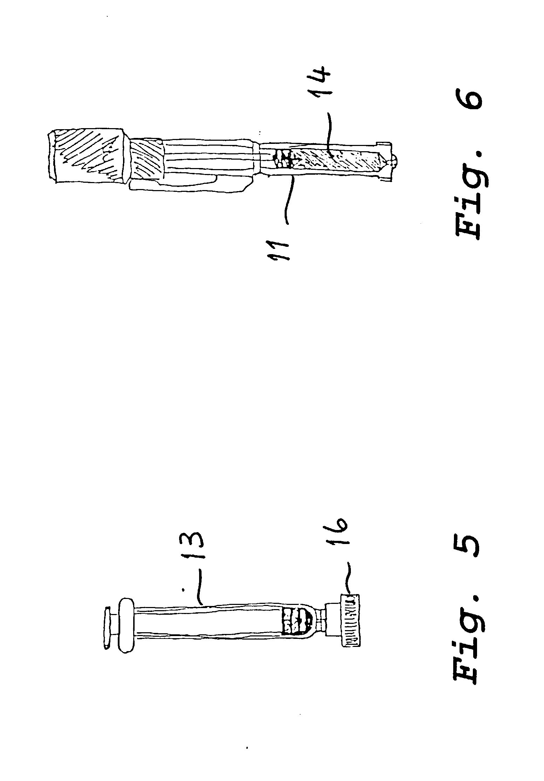

The next step is the detachment of the syringe 13 from the injector device 11, for instance by unscrewing the coupling member or adapter 16 and thereby separating it from the injector device 11. Figure 5 shows the syringe 13 and the coupling member or adapter 16 and Fig. 6 shows the injector device 11 after this operation.

After the step just described injector device 11 is ready to be operated in the way described in detail in the above mentioned International Patent Application WO 89/08469 for ejecting said medication 14 from the injector device and thereby pass the medication through the skin of the receiver of the injection.

A selected amount of medication injected in this way is preferably determined by the amount of medication 14 contained in the prefilled syringe 13. For this purpose, the entire amount of medication prefilled in the syringe 13 is transferred from the syringe 13 into the injector device 11 ,so that - in contrast to the known method of transfer described for example in the above mentioned International Patent Application WO 89/08469 - no measuring of the amount of medication by the patient is necessary.

## Claims

1. A method for parenterally administering a medication, comprising:
providing a needleless injector device (11) having a barrel portion, a gas storage portion, and an intermediate portion extending between the barrel portion and the gas storage portion;
providing a syringe (13) prefilled with a medication (14), said syringe being suitable for needleless loading of the injector device (11) with said medication prefilled in the syringe;
loading the medication (14) prefilled in the syringe (13) into a chamber (29) of the barrel portion of the injector device (11) through an aperture in the barrel portion thereof;
detaching the prefilled syringe (13) from the injector device (11); and
operating the loaded injector device (11) to eject the dosage of medication (14) contained therein from the injector device (11) and thereby pass said dosage of the medication through the skin of an injection receiver.

2. A method according to claim 1, wherein the step of loading the medication (14) into the injector device (11) includes:
connecting the prefilled syringe (13) to the injector device (11); thereby establishing a fluidic communication between the interior of the syringe (13) and the interior of the injector device (11); and
operating the prefilled syringe (13) for transferring the entire amount of medication prefilled in the syringe (13) from the syringe (13) into the injector device (11).

3. A needleless injection system, comprising:
a needleless injector device (11) that is dimensioned and arranged as an integral unit to be grasped in the hand of a user, the device (11) having a barrel portion, a gas storage portion, and an intermediate portion extending between the barrel portion and the gas storage portion; and
a syringe (13) prefilled with a medication (14), said syringe being suitable for loading the injector device (11) with said medication (14) prefilled in the syringe.

4. A system according to claim 3 further comprising coupling means (16) for connecting the prefilled syringe (13) to the needleless injector device (11) and thereby establishing a fluidic communication between the interior of the syringe (13) and the interior of the injector device (11) for the purpose of transferring a quantity of medication from the prefilled syringe (13) into the needleless injector device (11).
